# FASCICULE DE BREVET EUROPEEN

(11) **EP 3 063 539 B1**
(45) Date de publication et mention de la délivrance du brevet: **27.09.2017**
(21) Numéro de dépôt: 14796001.7
(22) Date de dépôt: 29.10.2014
(51) Int. Cl.: G01N 33/18, G01N 33/487, G01N 33/49, B29K 105/00, B29L 31/00, B29C 39/00, B29C 39/02, B29K 27/06, G01N 27/333

(54) **PROCEDE DE PREPARATION D'UNE MEMBRANE POLYMERIQUE D'UNE ELECTRODE A MEMBRANE POLYMERIQUE POUR LA DETECTION POTENTIOMETRIQUE D'AU MOINS UN ANALYTE PRESENT DANS UNE SOLUTION**
VERFAHREN ZUR HERSTELLUNG EINER POLYMERMEMBRAN EINER POLYMERMEMBRANELEKTRODE ZUR POTENTIOMETRISCHEN DETEKTION MINDESTENS EINES ANALYTEN IN EINER LÖSUNG
METHOD FOR PRODUCING A POLYMER MEMBRANE OF A POLYMER MEMBRANE ELECTRODE FOR THE POTENTIOMETRIC DETECTION OF AT LEAST ONE ANALYTE PRESENT IN A SOLUTION

(30) Priorité: 29.10.2013 FR 1360572
(43) Date de publication de la demande: 07.09.2016
(73) Titulaire: Commissariat à l'Énergie Atomique et aux Énergies Alternatives, 75015 Paris (FR)
(72) Inventeur: FILS, Julien, F-38210 Saint Quentin sur Isere (FR); CASTELLAN, Gaël, F-38360 Engins (FR); LAMBERT, Aurélien, F-74450 Saint-Jean-de-Sixt (FR)
(74) Mandataire: Brevalex
(86) Numéro de dépôt international: PCT/EP2014/073185
(87) Numéro de publication internationale: WO 2015/063129

(56) Documents cités:
- US-A- 5 607 567
- US-B1- 6 391 174

## Description

### DOMAINE TECHNIQUE

L'invention a trait à un procédé de préparation d'une membrane polymérique d'une électrode à membrane destinée à la détection quantitative potentiométrique d'au moins un analyte présent dans une solution.

On précise que, par analyte, on entend une espèce chimique présente en solution, qui peut être, plus spécifiquement, choisie parmi des ions ou encore des composés organiques d'intérêt (tels que l'urée, le glucose).

L'invention trouve donc application dans le domaine des capteurs chimiques comprenant des électrodes du type mentionné ci-dessus.

De tels capteurs, notamment lorsqu'ils sont destinés à la détection d'ions, sont utiles dans le domaine de l'environnement ou sanitaire, en particulier pour la gestion de la qualité de l'eau. Ils peuvent être notamment utilisés dans des laboratoires d'analyse de l'eau, afin de mesurer la concentration de certains ions, en vue d'accéder ainsi à la dureté de l'eau.

De tels capteurs peuvent également être utiles dans d'autres domaines, tels que :
- le domaine médical et plus spécifiquement le domaine du diagnostic, par exemple, pour l'analyse de constantes sanguines en vue de détecter certaines pathologies, comme une déficience rénale ;
- le domaine pharmaceutique ;
- le domaine de l'agriculture, en particulier, l'analyse de l'eau ; et
- le domaine industriel, tel que, par exemple, les centrales thermiques pour déterminer le degré d'encrassement de ces dernières, en particulier, au niveau des circuits de refroidissement.

### ETAT DE LA TECHNIQUE ANTÉRIEURE

A l'heure actuelle, de nombreuses méthodes ont été mises en place pour la détection d'analytes, tels que les ions, parmi lesquelles on peut citer :
- la détection par ionisation de flamme, dans laquelle l'éluat à analyser pénètre dans une flamme obtenue par combustion d'hydrogène et d'air, moyennant quoi il se forme des ions collectés par deux électrodes, entre lesquelles on applique une différence de potentiel, de laquelle résulte un courant électrique, qui est enregistré et mis en corrélation avec la quantité d'ions détectés ;
- la détection par photométrie de flamme, dans laquelle les ions à détecter sont soumis à la chaleur d'une flamme ce qui se caractérise par le passage de ces ions à un état excité ; le retour à l'état fondamental des électrons de la couche externe s'effectue avec émission lumineuse caractéristique des ions en présence, l'intensité de cette émission étant proportionnelle à la concentration desdits ions ;
- la détection par colorimétrie, dans laquelle les analytes à détecter sont mis en contact avec un réactif apte à réagir avec ceux-ci pour former un composé coloré, qui sera analysé par mesure d'absorbance pour remonter à la concentration desdits analytes.

Si ces méthodes font, d'une manière générale, preuve d'une bonne sensibilité, elles constituent toutefois des méthodes invasives, qui s'accompagnent d'une perturbation du milieu à analyser tant du point de vue chimique que, le cas échéant, du point de vue électrique.

Pour contrer ce type d'inconvénients, il est possible de recourir, notamment depuis les années 60, à une méthode de détection d'analytes par potentiométrie, dont le principe repose :
- d'une part, sur la mesure de la différence de potentiel, qui se développe entre deux électrodes (respectivement, une électrode indicatrice et une électrode de référence) en raison de l'activité de l'analyte présent dans l'échantillon ; et
- d'autre part, sur la détermination de l'activité de l'analyte par application de l'équation de Nernst en partant des valeurs de différence de potentiel.

Comme suggéré ci-dessus, un dispositif permettant de mettre en place cette méthode comprend au moins une cellule comprenant une électrode indicatrice et une électrode de référence qui plongent dans la solution à analyser, lesdites électrodes étant reliées à au moins une unité permettant de mesurer les variations de potentiel électrique et éventuellement de corréler ces variations à l'activité de l'analyte à détecter.

Si ces unités permettent de mesurer uniquement les variations de potentiel électrique, il peut être fait recours à un abaque préalablement réalisé qui permet de faire la corrélation entre les variations de potentiel électrique et l'activité de l'analyte contenu dans la solution.

L'électrode de référence est une électrode dont le potentiel E_{réf}, exactement connu, est indépendant de la concentration de l'analyte et toute autre espèce présente dans la solution à analyser. Classiquement, cette électrode de référence peut être une électrode au calomel ou une électrode Ag/AgCl.

L'électrode indicatrice est une électrode de mesure, qui développe, au contact de la solution à analyser, un potentiel E_{ind}, qui est fonction de l'activité de l'analyte.

Cette électrode peut se présenter sous forme d'une électrode à membrane, dont la membrane est sélective de l'analyte dont on souhaite déterminer la présence. Ces électrodes, lorsqu'elle est destinée à la détermination d'ions, peut être qualifiée d'électrode ion-sélective ou sélective aux ions (ce type d'électrodes étant connu sous la dénomination d'électrodes ISE pour « Ionic selective electrode »).

De façon générale, on distingue quatre types d'électrodes à membrane :
- les électrodes à membrane de verre ;
- les électrodes à membrane cristalline ;
- les électrodes à membrane liquide ; et
- les électrodes à membrane polymère.

Cette dernière catégorie tend à supplanter les autres catégories, car elles sont notamment plus souples que ne le sont les électrodes à membrane cristalline et elles sont plus robustes et nettement moins sensibles à la solubilisation que ne le sont les électrodes à membrane liquide.

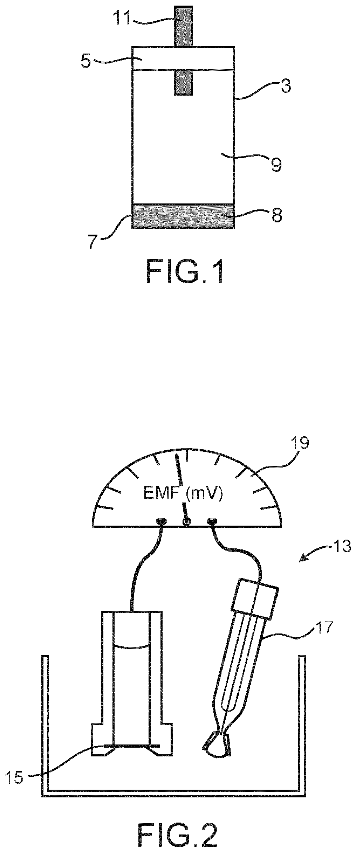

Ces électrodes à membrane, comme illustré sur la figure 1, se présentent classiquement sous la forme d'un tube (référencé 3), par exemple cylindrique, qui peut être en un matériau polymérique (tel qu'un matériau vinylique comme du polychlorure de vinyle) présentant une extrémité supérieure 5 et une extrémité inférieure 7, cette dernière étant destinée à être en contact avec la solution à analyser. Pour ce faire, l'extrémité inférieure 7 est obturée par une membrane polymérique dite sensible et sélective, car elle va être à même de capter préférentiellement un analyte d'une espèce particulière. En outre, le tube susmentionné présente une cavité interne 9 remplie d'une solution interne d'électrolyte (ou solution interne de remplissage ou plus simplement électrolyte) consistant classiquement en une solution aqueuse comprenant un sel, par exemple, un sel alcalin comme du NaCl, dans laquelle est plongé un élément de contact électrique 11 (cet élément pouvant être qualifié également d'électrode de contact, du fait qu'elle sert à « récupérer » le potentiel induit par la fixation de l'analyte sur la membrane).

Comme recensé dans l'article Faridbod et al., African Journal of Biotechnology Vol.6 (25), pp.2960-2987, les membranes polymériques sont réalisées selon deux grandes voies de synthèse.

La première voie de synthèse consiste à couler sur un support la solution précurseur de la membrane polymérique puis, après séchage, de découper à l'emporte-pièce la membrane polymérique, qui sera ensuite collée sur l'une des extrémités (celle destinée à être ultérieurement en contact avec la solution à analyser) d'un corps d'électrode. Cette voie de synthèse présente notamment l'inconvénient d'être d'une mise en oeuvre longue et coûteuse.

La deuxième voie de synthèse consiste, quant à elle, à plonger un corps d'électrode creux *via* l'une de ses extrémités (celle destinée à être ultérieurement en contact avec la solution à analyser) dans une solution précurseur de la membrane puis, après retrait, de laisser la solution déposée à ladite extrémité, moyennant quoi il subsiste la membrane polymérique.

Selon ces deux voies de synthèse, la solution interne d'électrolyte est introduite dans le corps d'électrode après formation de la membrane polymérique, ce qui peut générer un emprisonnement d'air entre la solution interne d'électrolyte et la membrane polymérique préalablement formée et ce qui entrave l'utilisation de l'électrode dans toutes les directions.

Les inventeurs se sont fixé pour but de proposer un nouveau procédé d'élaboration d'une membrane polymérique pour électrode à membrane ne présentant pas les inconvénients susmentionnés et notamment celui de la présence d'air entre la solution interne d'électrolyte et la membrane polymérique.

### EXPOSÉ DE L'INVENTION

Pour ce faire, l'invention a trait à un procédé d'élaboration d'une membrane polymérique d'une électrode à membrane polymérique destinée à la détection potentiométrique d'au moins un analyte présent dans une solution à analyser, ledit procédé comprenant les étapes successives suivantes :
a) une étape de dépôt d'une solution, en phase liquide, comprenant les constituants de la membrane polymérique à la surface d'une solution interne d'électrolyte, cette dernière étant en phase liquide et occupant la cavité interne d'un corps d'électrode ;
b) une étape de séchage de ladite solution comprenant les constituants de la membrane polymérique, moyennant quoi il subsiste à la surface de la solution interne d'électrolyte la membrane polymérique.

On précise que, par solution interne d'électrolyte, on entend la solution qui fait la jonction, au sein de la cavité interne de l'électrode, entre la membrane polymérique et l'élément de contact électrique. L'élément de contact électrique peut être une électrode métallique ou la grille d'un transistor, afin de former, par exemple, un transistor du type ISFET (correspondant à l'expression anglaise « Ion Selective Field Effect Transistor »). On peut également évoquer, au lieu de la terminologie de solution interne d'électrolyte, les terminologies suivantes : solution interne de remplissage, solution de référence interne ou encore plus simplement électrolyte.

On précise que, par surface d'une solution interne d'électrolyte, on entend, classiquement, la surface qui affleure à l'air libre à l'une des extrémités du corps d'électrode.

La membrane polymérique peut être, en particulier, une membrane polymérique ion-sélective (que l'on peut ainsi qualifier de membrane ISE), lorsque l'analyte est un ion.

Dans ce cas, la solution comprenant les constituants de la membrane peut comprendre :
- au moins un polymère en tant que tel, destiné à former une matrice polymérique ;
- au moins une substance active destinée à capter le ou les ions, constituant l'élément clé gouvernant la réponse de l'électrode ;
- au moins un solvant organique, par exemple, apolaire ; et
- éventuellement un ou plusieurs agents plastifiants utilisés, notamment, en vue de diminuer la température de transition vitreuse du ou des polymères et ainsi de les assouplir.

Le ou les polymères destinés à former la matrice polymérique peuvent être des polymères vinyliques, tels que le polychlorure de vinyle, des polysiloxanes, des polyuréthanes.

Pour préparer la solution comprenant les constituants de la membrane, on peut former un mélange, dit mélange initial, comportant les constituants de la membrane mentionnés ci-dessus, auquel on ajoute un solvant.

Le mélange initial peut comprendre une teneur en masse de polymère(s) allant de 20% et 50%, et le plus souvent, de 25% à 35% en masse par rapport à la masse totale du mélange initial. Un polymère adéquat peut être le polychlorure de vinyle.

Comme mentionné ci-dessus, le mélange intitial comprend la ou les substances actives destinées à capter le ou les ions, lesquelles comprennent :
- les composés ionophores ; et
- éventuellement les matériaux échangeurs d'ions, qui peuvent permettre d'ajuster la conductivité de la membrane.

La ou les substances actives peuvent être comprises à hauteur de 1 à 10 % en masse par rapport à la masse totale du mélange initial.

Des composés ionophores adaptés peuvent être des composés organiques formant cage, tels que des composés dits « couronnes ».

A titre d'exemple, on peut mentionner des composés calixarènes, tels que l'ester tétraéthylique d'acide 4-tert-butylcalix[4]arène-tétracétique, qui ont notamment la capacité de piéger les ions sodium Na⁺ (ce composé étant vendu par Sigma Aldrich sous la dénomination Ionophore X).

A titre d'exemple, on peut mentionner des composés du type « éthers couronnes », tels que le 2-dodécyl-2-méthyl-1,3-propanediyl bis[*N*-[5'-nitro(benzo-15-couronne-5)-4'-yl]carbamate], qui a notamment la capacité de piéger les ions potassium K⁺ (ce composé étant vendu par Sigma Aldrich sous la dénomination Ionophore III).

Le ou les agents plastifiants peuvent être choisis parmi les diesters de l'acide adipique tels que, par exemple, l'adipate de dioctyle (connu sous l'abréviation DOA).

Le ou les agents plastifiants peuvent être compris dans le mélange initial dans une proportion supérieure à celle du ou des polymères susmentionnés.

Le ou les agents plastifiants peuvent être compris à hauteur de 40 à 80 %, par exemple, de 55 à 65% en masse par rapport à la masse totale du mélange initial.

A ce mélange initial est ajouté un solvant organique, par exemple un solvant organique apolaire. Ce solvant peut être choisi parmi les éthers cycliques, tels que le tétrahydrofurane. La quantité ajoutée en masse de solvant peut être comprise entre 1 et 10 fois la masse du mélange initial.

Certains constituants de la solution comprenant les constituants de la membrane sont, avantageusement, non miscibles avec la solution interne d'électrolyte. Il s'agit notamment du ou des polymères, du ou des composés ionophores. Le ou les plastifiants peuvent être miscibles avec la solution interne d'électrolyte, sous réserve qu'ils présentent une affinité chimique supérieure vis-à-vis du ou des polymères que de la solution interne d'électrolyte. Ainsi, une fois déposée, la solution comprenant les constituants de la membrane reste à la surface de la solution interne d'électrolyte.

La solution comprenant les constituants de la membrane polymérique peut être préparée par un procédé comprenant les étapes suivantes :
- une étape de préparation du mélange initial mentionné ci-dessus;
- une étape d'ajout d'un solvant au mélange initial ; et
- une étape de mélange jusqu'à l'obtention d'une solution homogène.

Comme mentionné ci-dessus, l'étape a) consiste en une étape de dépôt d'une solution comprenant les constituants de la membrane polymérique à la surface d'une solution interne d'électrolyte, cette dernière étant en phase liquide et occupant la cavité interne d'un corps d'électrode.

Par phase liquide, on entend, généralement, dans ce qui précède et ce qui suit, une phase présentant une viscosité inférieure à 10000 cP.

Cette solution interne d'électrolyte peut être constituée d'une solution aqueuse, d'une solution comprenant de la glycérine, d'une solution comprenant un mélange comprenant de la glycérine et de l'eau ou elle peut être également un liquide ionique, étant entendu que cette solution interne d'électrolyte se présente sous forme liquide.

D'un point de vue pratique, cette étape a) peut consister à déposer une ou plusieurs gouttes de la solution comprenant les constituants de la membrane, par exemple, au moyen d'une pipette, à la surface de la solution interne d'électrolyte remplissant la cavité interne du corps d'électrode. Cette technique peut être ainsi qualifiée de « drop casting ».

Il s'entend que le volume de solution comprenant les constituants de la membrane polymérique sera choisi de sorte à occuper au moins la totalité de la surface de solution interne affleurant à l'une des extrémités du corps d'électrode, ce qui signifie, en d'autres termes, que le volume de la solution peut être choisi de sorte à déborder de la surface affleurante de solution interne.

Plus spécifiquement, la solution interne d'électrolyte peut être une solution aqueuse pouvant comprendre, en outre, un ou plusieurs sels, tels que des sels choisis, par exemple, parmi :
- les sels alcalins, comme le chlorure de sodium (NaCl), le chlorure de potassium (KCI) ;
- les sels alcalino-terreux comme le chlorure de calcium (CaCl₂).

Comme suggéré ci-dessus, il peut s'agir également d'une solution interne d'électrolyte comprenant au moins un solvant, qui est de la glycérine.

Une telle solution interne d'électrolyte peut comprendre un seul solvant, qui est de la glycérine (ce qui signifie, en d'autres termes, que la teneur en glycérine dans le solvant est de 100%) ou peut comprendre un mélange de solvants comprenant de la glycérine, par exemple, un mélange constitué de glycérine et d'eau.

Dans le cas d'un mélange de solvants, ce mélange de solvants peut comprendre de la glycérine, de préférence, à hauteur d'au moins 30% en masse par rapport à la masse totale du mélange de solvants, ce mélange de solvants comprenant, outre de la glycérine, de préférence, de l'eau.

De préférence encore, le mélange de solvants comprend de la glycérine à hauteur d'au moins 50%, avantageusement de 50% à 80%, et en particulier, de 60 à 70%, en masse par rapport à la masse totale du mélange de solvants.

Hormis la glycérine, une telle solution peut comprendre, en outre, un ou plusieurs sels choisis, par exemple, parmi :
- les sels alcalins, comme le chlorure de sodium (NaCl), le chlorure de potassium (KCI) ;
- les sels alcalino-terreux comme le chlorure de calcium (CaCl₂).

Le ou les sels peuvent être présents, au sein de la solution, à une concentration allant de 10⁻⁶ mol/L à une concentration de saturation, de préférence de 10⁻⁶ mol/L à 3 mol/L, par exemple de 100 mmol/L.

Une fois l'étape a) mise en oeuvre, le procédé de l'invention comprend une étape b) de séchage de ladite solution comprenant les constituants de la membrane polymérique, moyennant quoi il subsiste à la surface de la solution interne d'électrolyte la membrane polymérique.

De préférence, la membrane polymérique obture le corps d'électrode.

Cette étape de séchage peut consister à laisser la solution ainsi déposée à l'air libre (c'est-à-dire à température ambiante) pendant une durée suffisante pour permettre l'évaporation des constituants volatils (tels que le ou les solvants organiques) ou peut consister à placer l'ensemble dans une étuve chauffée, c'est-à-dire à une température suffisante (supérieure à la température ambiante extérieure à l'étuve) pour permettre l'évaporation des constituants volatils de la solution. Le séchage peut être effectué, également, par exposition à un rayonnement infrarouge. Ce mode de réalisation permet de chauffer principalement la membrane, tout en préservant l'électrolyte ou le corps de l'électrode, limitant ainsi les risques de dégradation.

En outre, le procédé peut comprendre, avant la mise en oeuvre de l'étape a), une étape de remplissage de la cavité interne d'un corps d'électrode par une solution interne d'électrolyte telle que définie ci-dessous.

Dans le cadre du procédé de l'invention, le corps d'électrode peut se présenter sous forme d'un corps creux (par exemple, un tube cylindrique creux ou une chambre parallélépipédique) dont une des extrémités (dite ci-dessous, première extrémité) est ouverte vers l'extérieur et dont la surface extérieure est délimitée par celle de solution interne d'électrolyte. Ce corps creux peut comprendre une autre extrémité, par exemple diamétralement opposée à la première extrémité, obturée par un élément de contact électrique, ladite solution interne d'électrolyte assurant la jonction entre ladite membrane et ledit élément de contact électrique.

Le corps d'électrode peut être en un matériau polymère, tel qu'un matériau polyvinylique (comme le polychlorure de vinyle), un matériau polyoléfinique (comme le polypropylène), un matériau polyméthacrylate (comme le polyméthacrylate de méthyle) ou un matériau polycarbonate.

L'élément de contact électrique peut être une tige métallique, dont une extrémité plonge dans la solution interne d'électrolyte et l'autre extrémité est reliée au circuit extérieur. Il peut également se présenter sous forme d'une pastille métallique, telle qu'une pastille en argent.

Ainsi, une fois la membrane polymérique obtenu, le produit résultant est une électrode à membrane, qui se présente sous forme d'un corps d'électrode dont la cavité interne est remplie d'une solution interne d'électrolyte, laquelle est en contact avec la membrane polymérique obtenue, qui obture le corps d'électrode.

Les membranes obtenues par le procédé de l'invention peuvent être des membranes de forme sphérique, pouvant présenter un diamètre allant de 0,5 mm à quelques cm, de préférence, entre 0,5 et 2 mm et pouvant présenter une épaisseur allant de 0,3 à 1 mm.

Le procédé de l'invention peut permettre l'obtention d'une membrane de faible surface, dont le diamètre est inférieur à 5 mm, voire même est inférieur à 1 mm. Il est particulièrement adapté à la réalisation d'électrodes de faible volume, lesquelles sont adaptées pour de faibles volumes d'échantillons et/ou à des dispositifs d'analyse compacts, par exemple, portables.

Comme suggéré ci-dessus, les électrodes à membrane polymérique sont destinées à la détection potentiométrique d'au moins un analyte présent dans une solution, ce qui signifie, en d'autres termes, que ces électrodes sont vouées à être intégrées dans des capteurs chimiques.

Ces capteurs chimiques pour la détection potentiométrique d'un analyte présente dans une solution, comprennent, classiquement, comme illustré sur la figure 2, au moins une cellule 13 comprenant :
- au moins une électrode à membrane polymérique 15 ;
- au moins une électrode de référence 17 ;
ces électrodes étant reliées à une unité 19 permettant de mesurer les variations de potentiel électrique entre l'électrode à membrane polymérique et l'électrode de référence.

Cette électrode de référence peut être classiquement une électrode au calomel ou une électrode Ag/AgCl.

Comme évoqué ci-dessus, le procédé de l'invention peut permettre la réalisation d'électrodes de faible surface. En outre, le procédé de l'invention peut présenter les avantages suivants :
- il est aisément industrialisable, le dépôt de la solution comprenant les constituants de la membrane pouvant être effectué à l'aide d'un automate de dispense d'un liquide ;
- il présente un nombre d'étapes limité ; et
- il est de mise en oeuvre simple.

D'autres caractéristiques et avantages de l'invention apparaîtront du complément de description qui suit qui se rapporte à un exemple de mise en oeuvre du procédé conforme à l'invention.

Bien entendu, ce complément de description n'est donné qu'à titre d'illustration de l'invention et n'en constitue en aucun cas une limitation.

### BREVE DESCRIPTION DES FIGURES

La figure 1 est une représentation en coupe transversale d'une électrode à membrane polymérique.
La figure 2 est une représentation en coupe transversale d'un capteur chimique comprenant une électrode à membrane polymérique.

### EXPOSÉ DÉTAILLÉ DE MODES DE RÉALISATION PARTICULIERS

### EXEMPLE 1

Dans cet exemple, il a été procédé à la préparation d'une membrane polymérique conforme à l'invention (dite première électrode).

Pour ce faire, un élément de petit volume sous forme d'une chambre parallélépipédique présentant une hauteur de 5 mm et deux faces opposées (dont l'une est ouverte sur l'extérieur avant formation de la membrane) de 4*4 mm de surface est remplie avec une solution interne d'électrolyte comprenant uniquement de la glycérine et du NaCl 100 mM jusqu'à affleurer jusqu'à la face ouverte de l'élément. Sur la surface de la solution interne affleurante, il est déposé une goutte de 500 µL la solution de membrane polymérique, laquelle est issue d'un mélange initial, auquel est ajouté un solvant.

Le mélange initial susmentionné comprend les ingrédients suivants :
- du polychlorure de vinyle à hauteur de 30% en masse par rapport à la masse totale du mélange initial ;
- un agent plastifiant spécifique : l'adipate de dioctyle (également dénommé DOA) à hauteur de 60% en masse par rapport à la masse totale du mélange initial;
- un composé ionophore calixarène spécifique : l'ester tétraéthylique d'acide 4-tert-butylcalix[4]arène-tétracétique vendu par Sigma-Aldrich sous la dénomination Ionophore X, ce composé étant présent à hauteur de 5% en masse par rapport à la masse totale du mélange initial;
- un composé conducteur ionique spécifique : le sel tétrakis(4-chlorophényl)borate tétradodécylammonium, ce composé étant présent à hauteur de 5% en masse par rapport à la masse totale du mélange initial.

A ce mélange initial est ajouté un solvant (le tétrahydrofurane), la masse ajoutée de solvant étant comprise entre une et deux fois la masse du mélange.

Cette solution polymérique n'est pas miscible avec la solution interne d'électrolyte.

La solution polymérique ainsi déposée est ensuite mise à sécher à l'air ambiant pendant 3 heures, moyennant quoi il subsiste une membrane présentant une épaisseur de 1 mm.

## Revendications

1. Procédé d'élaboration d'une membrane polymérique d'une électrode à membrane polymérique destinée à la détection potentiométrique d'au moins un analyte présent dans une solution à analyser, ledit procédé comprenant les étapes successives suivantes :
a) une étape de dépôt d'une solution, en phase liquide, comprenant les constituants de la membrane polymérique à la surface d'une solution interne d'électrolyte, cette dernière étant en phase liquide et occupant la cavité interne d'un corps d'électrode ;
b) une étape de séchage de ladite solution comprenant les constituants de la membrane polymérique, moyennant quoi il subsiste à la surface de la solution interne d'électrolyte la membrane polymérique.

2. Procédé selon la revendication 1, dans lequel la membrane polymérique est une membrane polymérique ion-sélective (que l'on peut ainsi qualifier de membrane ISE), lorsque l'analyte est un ion.

3. Procédé selon la revendication 1 ou 2, dans lequel la solution comprenant les constituants de la membrane polymérique comprend :
- au moins un polymère ;
- au moins une substance active destinée à capter le ou les analytes ;
- au moins un solvant organique ; et
- éventuellement un ou plusieurs agents plastifiants.

4. Procédé selon l'une quelconque des revendications précédentes, comprenant, avant la mise en oeuvre de l'étape a), une étape de remplissage de la cavité interne d'un corps d'électrode par une solution interne d'électrolyte telle que définie à la revendication 1.

5. Procédé selon l'une quelconque des revendications précédentes, dont l'étape b) est réalisée à température ambiante ou dans une étuve chauffée.

## Patentansprüche

1. Verfahren zur Herstellung einer Polymermembran einer Polymermembranelektrode zur potentiometrischen Detektion zumindest eines Analyten in einer zu analysierenden Lösung, wobei das Verfahren die nachstehenden, aufeinanderfolgenden Schritte umfasst:
a) einen Schritt des Aufbringens einer Lösung in flüssiger Phase mit den Bestandteilen der Polymermembran auf die Oberfläche einer inneren Elektrolytlösung, wobei letztere in flüssiger Phase vorliegt und den Innenhohlraum eine Elektrodenkörpers einnimmt;
b) einen Schritt des Trocknens der Lösung mit den Bestandteilen der Polymermembran, wodurch auf der Oberfläche der inneren Elektrolytlösung die Polymermembran bestehen bleibt.

2. Verfahren nach Anspruch 1, wobei die Polymermembran eine ionenselektive Polymermembran ist (die somit als ISE-Membran bezeichnet werden kann), wenn der Analyt ein Ion ist.

3. Verfahren nach Anspruch 1 oder 2, wobei die Lösung mit den Bestandteilen der Polymermembran enthält:
- zumindest ein Polymer;
- zumindest eine Wirksubstanz, die dazu bestimmt ist, den bzw. die Analyten zu ermitteln;
- zumindest ein organisches Lösungsmittel; und
- gegebenenfalls einen oder mehrere Weichmacher.

4. Verfahren nach einem der vorangehenden Ansprüche, umfassend vor dem Durchführen von Schritt a) einen Schritt des Füllens des Innenhohlraums eines Elektrodenkörpers mit einer inneren Elektrolytlösung, wie in Anspruch 1 definiert.

5. Verfahren nach einem der vorangehenden Ansprüche, wobei Schritt b) bei Raumtemperatur bzw. in einer Wärmekammer erfolgt.

## Claims

1. Method for making a polymer membrane of a polymer membrane electrode intended for the potentiometric detection of at least one analyte present in a solution to be analysed, said method including the following series of steps:
a) a step of depositing a solution, in the liquid phase, including the constituents of the polymer membrane at the surface of an internal electrolyte solution, the latter being in the liquid phase and occupying the internal cavity of an electrode body;
b) a step of drying said solution including the constituents of the polymer membrane, resulting in the polymer membrane at the surface of the internal electrolyte solution.

2. Method according to claim 1, wherein the polymer membrane is an ion-selective polymer membrane (which may thus be qualified as ISE membrane), when the analyte is an ion.

3. Method according to claim 1 or 2, wherein the solution including the constituents of the membrane includes:
- at least one polymer;
- at least one active substance intended to capture the analyte(s);
- at least one organic solvent; and
- optionally, one or more plasticizing agents.

4. Method according to any one of the previous claims, including, before the implementation of step a), a step of filling the internal cavity of an electrode body with an internal electrolyte solution as defined in claim 1.

5. Method according to any one of the previous claims, wherein step b) is performed at room temperature or in a heated oven.
